# EUROPÄISCHE PATENTSCHRIFT

(11) **EP 0 347 868 B1**
(45) Veröffentlichungstag und Bekanntmachung des Hinweises auf die Patenterteilung: **10.01.1996**
(21) Anmeldenummer: 89111265.8
(22) Anmeldetag: 21.06.1989
(51) Int. Cl.: A61B 10/00, G01N 35/02

(54) **Vorrichtung zum Abfangen von einer Gewebeprobe, Gewebeflüssigkeit od.dgl.**
Device to catch a tissue sample, lymph or the similar
Dispositif pour recueillir un échantillon de tissus, la lymphe ou l'équivalent

(30) Priorität: 24.06.1988 DE 3821336; 14.04.1989 DE 3912257
(43) Veröffentlichungstag der Anmeldung: 27.12.1989
(73) Patentinhaber: Kothe, Lutz, D-78315 Radolfzell (DE)
(72) Erfinder: Kothe, Lutz, D-78315 Radolfzell (DE)
(74) Vertreter: Weiss, Peter, Dr. rer.nat.

(56) Entgegenhaltungen:
- EP-A- 0 119 405
- EP-A- 0 270 363
- EP-A- 0 279 358
- WO-A-83/01119
- FR-A- 2 236 474
- US-A- 4 647 432

## Beschreibung

Die Erfindung betrifft eine Vorrichtung zum Abfangen von einer Gewebeprobe, Gewebeflüssigkeit od. dgl., welche von einem chirurgischen Instrument abgetrennt und/oder angesaugt wird, wobei zwischen dem chirurgischen Instrument und einer Auffangvorrichtung eine Schlauchleitung besteht, welcher eine vakuumerzeugende Einrichtung zugeordnet ist.

In der EP-A-0 119 405 wird eine medizinische Vorrichtung mit einer Schneidzangeneinrichtung zum Entnehmen von Gewebeproben, Entfernen von langgestreckten Körperelementen, wie Nerven- oder Venenabschnitten, od. dgl. beschrieben, wobei ein Innenrohr in ein Außenrohr eingesetzt ist.

Durch die relative Bewegung von Innenrohr zu Außenrohr wird eine Schneidzangeneinrichtung bedient, durch welche entsprechende Gewebeproben abgeschnitten werden. Diese medizinische Vorrichtung steht über einen entsprechenden Schlauch mit einer Auffangeinrichtung in Verbindung, wobei die entnommenen Gewebeproben durch den Aufbau eines Vakuums angesaugt werden.

Aufgabe der vorliegenden Erfindung ist es, eine in der oben genannten Europäischen Patentanmeldung nur angedeutete Vorrichtung zum Abfangen von einer Gewebeprobe, Gewebeflüssigkeit od. dgl. näher zu umschreiben und besonders bevorzugte Ausführungsformen erfinderisch auszugestalten. Diese Vorrichtung soll kostengünstig herstellbar und einfach zu bedienen sein.

Zur Lösung dieser Aufgabe führt, daß die Schlauchleitung in eine Kammer in einem Übertragungskopf einmündet, welcher mit einer Ringdichtung der Auffangvorrichtung aufsitzt, wobei die Kammer über eine Leitung mit der vakuumerzeugenden Einrichtung in Verbindung steht.

Hierdurch wird das eigentliche chirurgische Instrument von der erfindungsgemäßen Vorrichtung separiert. In der Praxis hat sich erwiesen, daß eine dauernde Zuordnung des chirurgischen Instrumentes bzw. der Schlauchleitung zu der Auffangeinrichtung nicht günstig ist, da durch das Vakuum nicht nur gewünschte Gewebeproben, sondern auch beispielsweise Gewebeflüssigkeit angesaugt wird. Zur Trennung von gewünschten und ungewünschten Entnahmen ist in einem Ausführungsbeispiel vorgesehen, daß der Übertragungskopf mit einer Drehventileinheit verbunden ist.

Mittels dieser Drehventileinheit ist die Verbindung von Übertragungskopf zur vakuumerzeugenden Einrichtung herstell- und unterbrechbar.

Für die Drehventileinheit sind eine Reihe von Ausführungsformen denkbar und sollen von dem vorliegenden Erfindungsgedanken umfaßt werden. Bevorzugt besteht die Drehventileinheit in einer einfachen Ausführung aus einer Grundscheibe und einer mit dem Übertragungskopf verbundenen Drehscheibe, welche zur Grundscheibe hin eine Achse besitzt. Die Grundscheibe wiederum weist einen Anschluß zu einer Leitung zur Vakuumpumpe hin auf. Die Drehscheibe dagegen besitzt zumindest zwei Bohrungen, welche alternierend mit dem Anschluß bzw. einer Bohrung in der Grundscheibe in Verbindung bringbar sind. Durch die eine Bohrung wird der Übertragungskopf mit der Vakuumpumpe gekoppelt, durch die andere Bohrung erfolgt eine Verbindung über einen Schlauch zu einem separaten Auffangbehältnis. Dieses separate Auffangbehältnis dient dem Auffangen von nicht gewünschten Entnahmen, beispielsweise Gewebeflüssigkeit. Hierzu ist das Auffangbehältnis wiederum uber einen Schlauch mit dem Übertragungskopf in Verbindung zu bringen, je nachdem, in welcher Gebrauchslage sich der Übertragungskopf befindet.

Der Übertragungskopf selbst ist im übrigen über einen Drehhebel mit der Drehventileinheit verbunden. Dieser Drehhebel wird von einer Längsbohrung durchsetzt, welche einerseits eine Verbindung zur Vakuumpumpe besitzt und andererseits in eine in dem Übertragungskopf gebildete Kammer einmündet.

Diese Kammer wird teilweise von einer Hülse durchsetzt, welche wiederum mit dem Schlauch in Verbindung steht, der zu dem chirurgischen Instrument führt.

Die Hülse besitzt eine Hülsenöffnung und ist mit dieser Öffnung auf eine Anschlußdichtung zu dem Schlauch zu dem Auffangbehältnis in Verbindung zu bringen.

Somit ist es auf einfache Art und Weise möglich, den Übertragungskopf bei einer gewünschten Entnahme der Auffangeinrichtung aufzusetzen, während er bei einer ungewünschten Entnahme mit dem Auffangbehältnis in Verbindung zu bringen ist, wobei in beiden Fällen ein Vakuum aufgebaut wird, dessen Zuordnung zu einer der beiden Tätigkeiten allein durch die Drehventileinheit bzw. das Drehen des Übertragungskopfes erfolgt.

Bevorzugt soll die Kammer belüftbar sein, damit das Vakuum sowohl beim Abheben des Übertragungskopfes von der Auffangeinrichtung wie auch von der Verbindung zu dem Auffangbehältnis unterbrochen wird. Damit wird vermieden, daß in dieser Gebrauchslage gewünschte oder ungewünschte Entnahmen stattfinden.

In einem bevorzugten Ausführungsbeispiel besitzt hierzu die Kammer im Übertragungskopf eine Bohrung, welche von einem Hebel verschließbar ist. Über diesen Hebel wird der Übertragungskopf angehoben, so daß gleichzeitig mit dem Anheben auch ein Belüften der Kammer stattfindet.

In einem anderen Ausführungsbeispiel der Erfindung kann der Übertragungskopf aus einem Hauptgehäuse bestehen, welches eine Innenkammer umschließt, in die ein Anschlußstutzen für eine Vakuumleitung einmündet.

Zur Entlüftung dieser Innenkammer sind Bohrungen bzw. Luftöffnungen in dem Gehäuse vorgesehen, welche beispielsweise über eine Schubhülse und eine entsprechende Dichtung verschlossen werden können.

Die Verbindung zwischen Übertragungskopf und Schlauchleitung zu dem chirurgischen Instrument sollte so gestaltet sein, daß sie leicht lösbar ist. Hierdurch erleichtert sich ein Austausch von unterschiedlichen chirurgischen Instrumenten oder auch dann, wenn beispielsweise die Schlauchleitung durch eine Gewebeprobe verstopft ist. Erfindungsgemäß mündet deshalb die Schlauchleitung in ein Anschlußstück ein, welches eine Rastverbindung mit dem Übertragungskopf eingeht. Der Einfachheit halber besteht die Rastverbindung aus einer segmentierten Ringhülse am Übertragungskopf, welcher in sich einen Kugelsitz ausbildet. In diesen Kugelsitz kann eine Kugel des Anschlußstückes einrasten, wobei diese Kugel zur Innenkammer hin noch mit einer Ringdichtung belegt ist. Wird in der Innenkammer des Übertragungskopfes ein Vakuum aufgebaut, so zieht dieses Vakuum die Kugel gegen die Ringdichtung und drückt beide gegen den Kugelsitz in der Ringhülse.

Auch für die Auffangeinrichtung hat sich der Erfinder Neuerungen einfallen lassen. In dem bevorzugten Ausführungsbeispiel besteht die Auffangeinrichtung aus einem Stützkörper und einem diesem aufgesetzten Drehteller. In dem Stützkörper soll ein Antrieb für den Drehteller vorgesehen sein, wobei dieser Antrieb beispielsweise aus einem pneumatisch betätigbaren Schrittmotor besteht.

Die Lagebestimmung des Drehtellers bzw. dessen Fixierung geschieht über entsprechende Rastkugeln zwischen dem Stützkörper und dem Drehteller. Diese Rastkugeln sind der Einfachheit halber federgelagert.

Der Drehteller selbst wiederum besteht aus einer Tragscheibe und einer Deckscheibe zwischen denen die Kragen von Reagenzgläsern eingeklemmt werden. In diesem Fall besitzt die Tragscheibe entsprechende Durchstecköffnungen für die Reagenzgläser. Selbstverständlich ist, daß die Tragscheibe von der Deckscheibe abnehmbar und damit die Reagenzgläser entnehmbar ausgestaltet sind.

Bevorzugt soll der ganze Drehteller vom Stützkörper abnehmbar ausgestaltet sein, wozu er der Einfachheit halber auf eine Antriebswelle od. dgl. aufgesetzt ist.

Die gesamte Vorrichtung ist möglichst einfach aufgebaut und damit kostengünstig herstellbar. Sie unterliegt keinerlei Verschleiß und kann mit Hilfe des drehbaren Übertragungskopfes auf einfache Weise von der einen in eine andere Gebrauchslage versetzt werden.

Im Bedarfsfall können alle Bewegungsabläufe automatisiert oder halbautomatisiert werden. Hierzu hat der Erfinder ebenfalls eine Vorrichtung entwickelt, bei der die entsprechende Auffangvorrichtung nicht auf einem Kreis sondern linear angeordnet sind. In der Regel dürften die Auffangvorrichtungen in diesem Fall aus Magazinen für Reagenzgläser bzw. Sekretflaschen bestehen.

Dies Magazine sind in einem Schacht eines Gehäuses angeordnet, dem eine Führungsschiene für einen Schlitten zugeordnet ist, welcher den Übertragungskopf trägt. Über entsprechende Sensoren werden die Positionen des Übertragungskopfes gesteuert und den entsprechenden Reagenzgläsern oder Sekretflaschen zugeordnet.

Der Schlitten mit dem Übertragungskopf wird bevorzugt motorisch angetrieben, wobei hier jede handwerkliche Ausgestaltung denkbar ist.

Zur Automatisierung gehört auch, daß die Innenkammer des Übertragungskopfes automatisch belüftbar ist. Hierzu bietet sich vor allem die oben erwähnte Schubhülse an, welche elektromotorisch angehoben werden kann, wodurch die Luftöffnungen zur Innenkammer freigegeben werden. Auch hier sind aber andere Ausgestaltungen denkbar.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
- Fig. 1: eine schematisch dargestellte Draufsicht auf eine erfindungsgemäße Vorrichtung zum Abfangen von Gewebeproben od. dgl.,
- Fig. 2: eine schematisch dargestellte Draufsicht auf die Vorrichtung gemäß Fig. 1 in einer weiteren Gebrauchslage;
- Fig. 3: einen vergrößert dargestellten Querschnitt durch einen Übertragungskopf der Vorrichtung gemäß den Figuren 1 und 2;
- Fig. 4: eine teilweise gebrochen dargestellte Seitenansicht einer Drehventileinheit;
- Fig. 5: einen vergrößert dargestellten Querschnitt durch eine Auffangeinrichtung der Vorrichtung gemäß den Figuren 1 und 2.
- Fig. 6: eine perspektivische Ansicht einer weiteren Ausführungsform einer erfindungsgemäßen Vorrichtung zum Abfangen von Gewebeproben od. dgl.,
- Fig. 7: einen schematisch dargestellten Querschnitt durch eine erfindungsgemäße Vorrichtung ähnlich Fig. 6;
- Fig. 8: einen vergrößert dargestellten Querschnitt durch einen Teilbereich der Erfindung gemäß den Figuren 6 und 7;
- Fig. 9: eine Draufsicht auf einen Teil eines Übertragungskopfes.

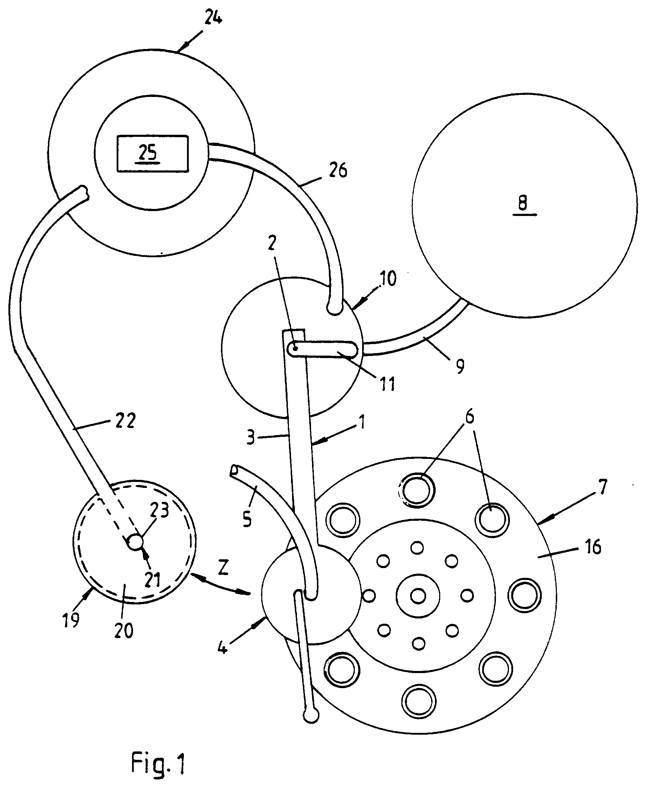

Eine erfindungsgemäße Vorrichtung zum Abfangen von Gewebeproben od. dgl. weist gemäß Figur 1 einen Dreharm 1 auf, welcher um eine Drehachse 2 in Richtung z drehbar ist. Dieser Dreharm 1 besteht im wesentlichen aus einem Drehhebel 3 sowie einen Übertragungskopf 4.

In den Übertragungskopf 4 mündet eine Schlauchleitung 5 ein, welche, nicht näher dargestellt, mit einer entsprechenden Schneidzangeneinrichtung verbunden ist, wie sie beispielsweise in dem Deutschen Gebrauchsmuster G 87 02 446 aufgezeigt ist.

Durch diesen Übertragungskopf 4 gelangt eine Gewebeprobe aus der Schlauchleitung 5 in entsprechende Reagenzgläser 6 einer Auffangeinrichtung 7.

Zum Ansaugen der Gewebeproben besitzt der Dreharm 1 einen Anschluß an eine entsprechende Vakuumpumpe 8. Dieser Anschluß wird durch die Leitung 9 angedeutet. Diese Leitung 9 mündet in eine Drehventileinheit 10, welche über ein Leitungsstück 11 mit dem Dreharm 1 in Verbindung steht.

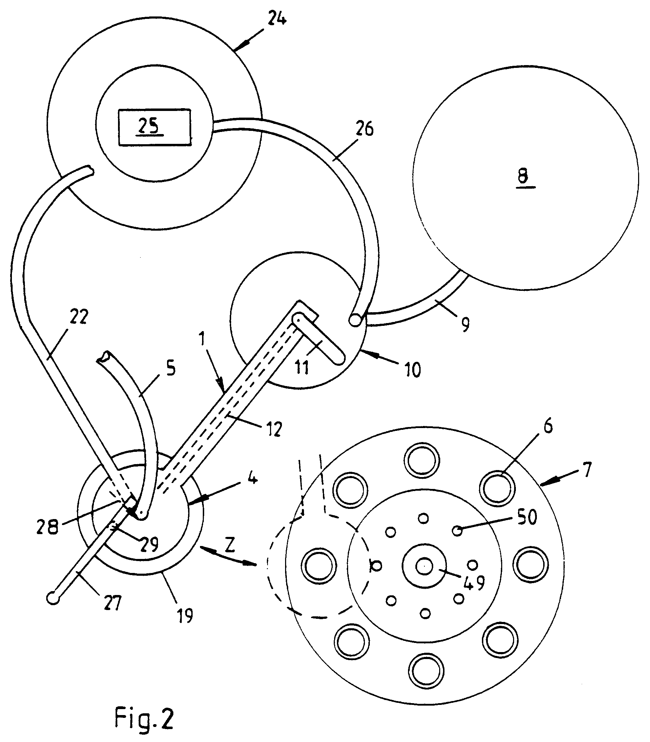

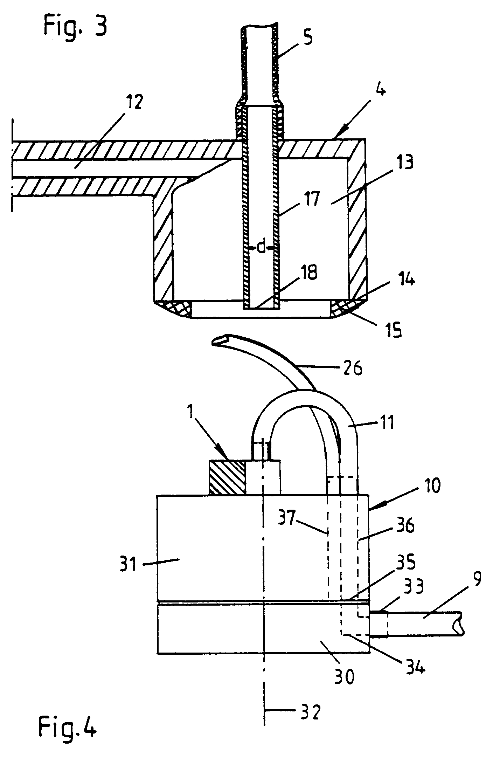

Hierzu besitzt das Leitungsstück 11 eine Verbindung zu einer in Figur 2 gestrichelt dargestellten Längsbohrung 12, welche, wie insbesondere in Figur 3 verdeutlicht wird, in einer Kammer 13 in den Übertragungskopf 4 ausmündet.

Der Umfangsrand 14 des Übertragungskopfes 4 ist mit einer Ringdichtung 15 belegt, welche nach Figur 1 einer Scheibe 16 aufsitzt und dabei ein Reagenzglas 6 umfängt. In dieser Gebrauchslage kann über die Vakuumpumpe 8 in der Kammer 13 ein Vakuum aufgebaut werden, so daß Gewebeproben durch die Schlauchleitung 5 angesaugt werden. Die Schlauchleitung 5 ist dabei einer Hülse 17 aufgesetzt, welche den größten Teil der Kammer 13 durchquert.

Das Vakuum ist dabei so aufgebaut, daß es bei dem relativ geringen Durchmesser d der Hülse 17 bzw. der Schlauchleitung 5 zum Ansaugen der Gewebeprobe genügt, daß diese aber nach Austritt aus der Hülsenöffnung 18 in das entsprechende Reagenzglas 6 fallen kann. Durch das entsprechende Volumen der Kammer 13 wird dieses Vakuum beim Austritt der Gewebeprobe aus der Hülsenöffnung 18 im wesentlichen aufgehoben.

In vielen Anwendungsfällen ist es wünschenswert, daß vor dem eigentlichen Ansaugen von Gewebeproben beispielsweise ein Absaugen von Gewebeflüssigkeit stattfindet bzw. kein Vakuum in Verbindung mit dem Füllen eines Reagenzglases 6 aufgebaut werden soll. Zu diesem Zweck befindet sich der Dreharm 1 in der in Figur 2 gezeigten Gebrauchslage. Dabei sitzt der Übertragungskopf 4 einem Behältnis 19 auf, das mit einem Deckel 20 verschlossen ist. Diesen Deckel 20 durchgreift ein Anschlußnippel 21, dem im Inneren des Behältnisses 19 ein Schlauch 22 aufgeschoben ist. Andererseits besitzt dieser Anschlußnippel eine Anschlußdichtung 23, welche bei der in Figur 2 gezeigten Gebrauchslage die Hülse 17 im Bereich der Hülsenöffnung 18 umfängt.

Der Schlauch 22 mündet vom Behältnis 19 kommend in ein weiteres geschlossenes Auffangbehältnis 24 ein. In diesem Auffangbehältnis 24 ist ein Druckmeßgerät 25 vorgesehen, über welches der entsprechende Vakuumdruck ermittelt werden kann. Ferner steht dieses Druckmeßgerät 25 über einen Schlauch 26 mit der Drehventileinheit 10 in Verbindung.

Dabei ist in der in Figur 2 gezeigten Gebrauchslage mittels dieser Dreheinheit 10 eine Verbindung zwischen dem Schlauch 26 und der Leitung 9 zur Vakuumpumpe 8 hergestellt. Hierdurch wird in dem Auffangbehältnis 24 ein Vakuum erzeugt, über welches entsprechende Gewebeflüssigkeit od. dgl. durch den Schlauch 22 aus der Schlauchleitung 5 angesogen wird. Dadurch, daß die Hülse 17 mit ihrer Hülsenöffnung 18 direkt der Anschlußdichtung 23 aufsitzt, findet in dem Übertragungskopf 4 kein Vakuumabfall statt, so daß die Flüssigkeit ohne Unterbrechung in das Auffangbehältnis 24 eingesaugt werden kann.

Zum Drehen des Dreharmes 1 in Drehrichtung z ist ferner dem Übertragungskopf 4 ein Hebel 27 zugeordnet. Dieser Hebel 27 bildet mit dem Übertragungskopf 4 ein Drehgelenk 28, welches in Figur 2 nur strichpunktiert dargestellt ist. In Gebrauchslage verschließt der Hebel 27 eine Bohrung 29, welche zur Kammer 13 führt. Wird der Hebel 27 angehoben, so wird auch die Kammer 13 über diese Bohrung 29 belüftet und das Vakuum egalisiert.

Die Drehventileinheit 10 besteht der Einfachheit halber aus einer Grundscheibe 30 und einer Drehscheibe 31.

Diese Drehscheibe 31 ist im Verhältnis zur Grundscheibe 30 um eine Achse 32 drehbar. Die Grundscheibe 30 steht über einen entsprechenden Anschluß 33 mit der Leitung 9 zur Vakuumpumpe 8 in Verbindung. Ferner wird die Grundscheibe 30 von einer entsprechenden Bohrung 34 durchquert, welche eine Bohrungsöffnung 35 zur Drehscheibe 31 hin besitzt. Dabei ist die Drehscheibe 31 von zwei achsparallelen Bohrungen 36 und 37 durchsetzt, wobei die achsparallele Bohrung 36 einen Anschluß zu dem Leitungsstück 11 besitzt, während die achsparallele Bohrung 37 mit dem Schlauch 26 in Verbindung steht.

Je nach Drehung der Drehscheibe 31 wird entweder die achsparallele Bohrung 36 oder die achsparallele Bohrung 37 mit der Bohrung 34 in Verbindung gebracht. Hierdurch wird entweder eine Verbindung zwischen der Vakuumpumpe 8 und der Kammer 13 oder der Vakuumpumpe 8 und dem Auffangbehältnis 24 hergestellt. Je nach gewünschter Gebrauchslage wird somit entweder Gewebeflüssigkeit in das Auffangbehältnis 24 oder aber abgeschnittene Gewebeproben in ein Reagenzglas 6 abgeführt.

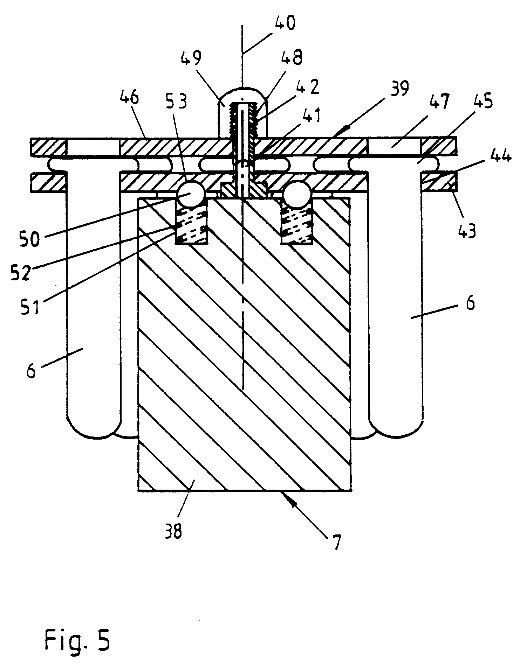

Die Auffangeinrichtung 7 weist gemäß Figur 5 einen Stützkörper 38 auf, welcher, der Übersichtlichkeit halber nicht dargestellt, ggfs. einen beispielsweise pneumatischen Antrieb für einen Drehteller 39 besitzt. Dieser Drehteller 39 dreht dabei um eine Drehachse 40, wobei eine entsprechende Antriebswelle bei 41 lediglich angedeutet ist. Diese Antriebswelle 41 greift als Stift in eine Verbindungshülse 42 ein, die wiederum mit einer Tragscheibe 43 verbunden ist.

Die Tragscheibe 43 besitzt Durchstecköffnungen 44 für Reagenzgläser 6, wobei diese Reagenzgläser 6 mit ihrem Kragen 45 der Tragscheibe 43 aufliegen. Darüber ist eine entsprechende Deckscheibe 46 vorgesehen, welche wiederum entsprechende Öffnungen 47 zu den Reagenzgläsern 6 hin besitzt. In Gebrauchslage werden diese Öffnungen 47 von der Ringdichtung 15 umfangen.

Die Verbindungshülse 42 durchgreift ebenfalls die Deckscheibe 46 und besitzt nach Durchgriff ein Außengewinde 48. In Gebrauchslage ist diesem Außengewinde 48 ein Schraubkopf 49 aufgesetzt, welcher dem Zusammenhalt des Drehtellers 39 dient.

Sollten beispielsweise alle Reagenzgläser 6 gefüllt sein, so braucht nur der gesamte Drehteller 39 von der Antriebswelle 41 abgezogen und durch einen neuen ersetzt zu werden.

Der Ausrichtung der Reagenzgläser 6 bzw. der Öffnungen 47 im Verhältnis zum Übertragungskopf 4 dienen entsprechende Rastkugeln 50, welche in Blindbohrungen 51 des Stützkörpers 38 auf Schraubenfedern 52 aufsitzen. Andererseits greifen diese Rastkugeln 50 in entsprechende Rastmulden 53 der Tragscheibe 43 ein. Auf diese Weise wird der Drehteller 39 getaktet. Andere Rastorgane zum Takten sind ebenfalls möglich und werden vom Erfindungsgedanken umfaßt. Dieses Takten kann sowohl automatisch wie auch halbautomatisch geschehen.

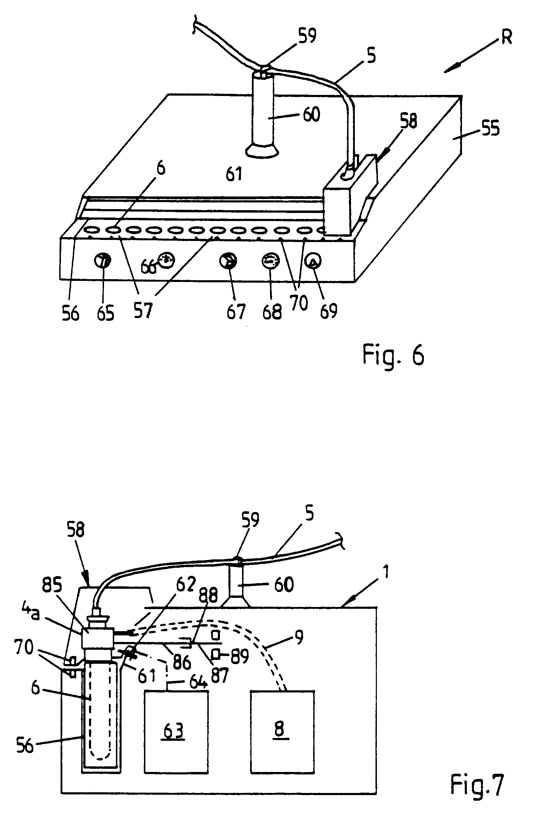

In den Figuren 6 und 7 ist eine automatische Vorrichtung zum Abfangen von Gewebeproben, Gewebeflüssigkeit od. dgl. dargestellt.

Hierzu weist diese Vorrichtung R ein Gehäuse 55 auf, welches frontwärtig einen Schacht 56 für in Reihe angeordnete Magazine 57 zur Aufnahme von Reagenzgläser 6 besitzt. Diese Magazine 57 können von einem Schlitten 58 überfahren werden, in den der Übertragungskopf 4a (siehe Figur 7) eingesetzt ist. Von diesem Übertragungskopf 4a führt die Schlauchleitung 5 zu dem nicht näher dargestellten medizinischen Gerät zum Abschneiden einer Gewebeprobe. Im vorliegenden Ausführungsbeispiel ist diese Schlauchleitung 5 durch eine Klammer 59 an einem Schlauchturm 60 gehalten, der wiederum dem Gehäuse 55 aufsitzt.

Der Schlitten 58 wird entlang einer mit dem Gehäuse 55 verbundenen Führungsschiene 61 bewegt, wozu beispielsweise diese Führungsschiene 61 beidseits von Rollen 62 angegriffen wird. Der Antrieb des Schlittens 58 erfolgt mittels eines Transportmotors 63, der in Figur 7 nur schematisch angedeutet ist. Gleichfalls ist auch die getriebemäßige Verbindung 64 zwischen dem Transportmotor 63 und dem Schlitten 58 nur schematisch strichpunktiert dargestellt.

Im vorliegend gezeigten Ausführungsbeispiel 4a des Übertragungskopfes mündet die Vakuumleitung 9, die von der Vakuumpumpe 8 kommt, direkt in den Übertragungskopf 4a selbst ein. Dieser Übertragungskopf 4a wird weiter unten näher beschrieben.

An der Frontseite der Vorrichtung R ist ein Ein- und Ausschalter 65, ein Vakuumdosierregler 66, ein Transportschalter 67, ein Reagenzglaszähler 68 sowie ein Schalter 69 für das Abfüllen von beispielsweise einer Sekretflasche vorgesehen.

Die Steuerung der Bewegung des Schlittens 58 bzw. dessen Halt über einem entsprechenden Reagenzglas 6 erfolgt über beispielsweise Sensoren 70, welche Annäherungs- bzw. Magnetschalter sein können.

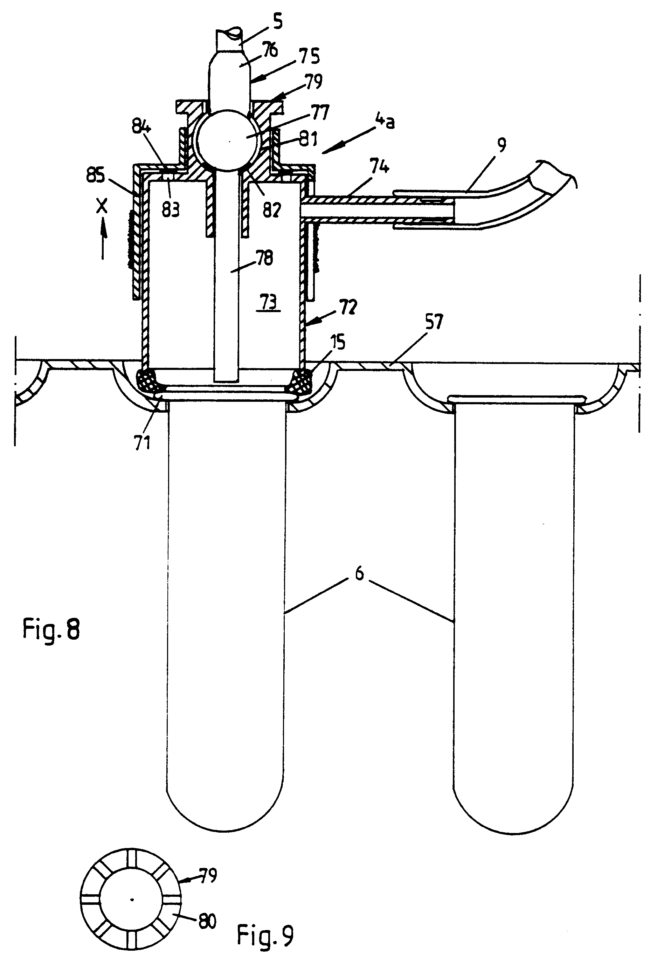

In Figur 8 ist vergrößert dargestellt, wie die Reagenzgläser 6 in ein Magazin 57 eingesetzt sind. Auf den oberen Rand 71 eines Reagenzglases 6 ist ein Übertragungskopf 4a aufgesetzt. Der Abdichtung im Aufsetzbereich dient die Ringdichtung 15.

Der Übertragungskopf 4a besitzt ein Hauptgehäuse 72, welches eine Innenkammer 73 umschließt. Diese Innenkammer 73 steht über einen Anschlußstutzen 74 mit der Vakuumleitung 9 in Verbindung und kann so evakuiert werden.

Im Kopfbereich ist in das Hauptgehäuse 72 ein Anschlußstück 75 für die Schlauchleitung 5 eingesetzt. Dabei steckt die Schlauchleitung 5 in einem Einsatzstück 76, welches mit einer Kugel 77 verbunden ist. Aus dieser Kugel 77 mündet dann ein Röhrchen 78 aus, durch welches die entnommene Gewebeprobe in das Reagenzglas 6 fällt. Es versteht sich von selbst daß sich in dem Anschlußstück 75 ein axialer Durchgang durch das Einsatzstück 76, die Kugel 77 und das Röhrchen 78 befindet.

Die Verbindung zwischen dem Anschlußstück 75 und dem Hauptgehäuse 72 wird durch ein Einsetzen der Kugel 77 in eine segmentierte Ringhülse 79 bewirkt.

Diese segmentierte Ringhülse 79 besteht, wie insbesondere in Figur 9 gezeigt, aus einzelnen Segmenten 80, welche sich nach außen aufbiegen können und so den Durchlaß der Kugel 77 in einen Kugelsitz 81 gestatten. In dem Kugelsitz 81 befindet sich auch eine Dichtung 82, gegen welche bei Aufbau eines Vakuums innerhalb der Innenkammer 73 die Kugel 77 gedrückt wird.

Neben der Ringhülse 79 befinden sich Luftöffnungen 83, vergleichbar mit den Bohrungen 29 in dem Hauptgehäuse 72. In Gebrauchslage sind diese Luftöffnungen 83 von einer Dichtung 84 verschlossen. Diese Dichtung 84 ist mit einer Schubhülse 85 verbunden, welche zumindest teilweise das Hauptgehäuse 72 umfängt. Wird diese Schubhülse 85 in Richtung x verschoben, so werden auch die Luftöffnungen 83 freigegeben und die Kammer 73 kann belüftet werden.

In der automatisierten Ausführungsform der Erfindung gemäß den Figuren 6 und 7 geschieht diese Bewegung der Schubhülse 85 automatisch über ein Gestänge 86, welches von einem Bewegungsarm 87 angegriffen wird. Die entsprechende Drehung des Bewegungsarms 87 um eine Drehachse 88 wird durch einen elektromagnetischen Antrieb 89 bewirkt.

## Patentansprüche

1. Vorrichtung zum Abfangen von einer Gewebeprobe, Gewebeflüssigkeit od. dgl., welche von einem chirurgischen Instrument abgetrennt und/oder angesaugt wird, wobei zwischen dem chirurgischen Instrument und einer Auffangvorrichtung (7, 57) eine Schlauchleitung (5) besteht, welcher eine vakuumerzeugende Einrichtung (8) zugeordnet ist,
dadurch gekennzeichnet,
daß die Schlauchleitung (5) in eine Kammer (13, 73) in einem Übertragungskopf (4, 4a) einmündet, welcher mit einer Ringdichtung (15) der Auffangvorrichtung (7, 57) aufsitzt, wobei die Kammer (13, 73) über eine Leitung (9, 11, 12) mit der vakuumerzeugenden Einrichtung (8) in Verbindung steht.

2. Vorrichtung nach Anspruch 1, dadurch gekennzeichnet, daß die Kammer (13, 73) belüftbar ist.

3. Vorrichtung nach Anspruch 2, dadurch gekennzeichnet, daß eine Bohrung (29, 83) zum Belüften der Kammer (13, 73) von einem Hebel (27) überdeckt ist.

4. Vorrichtung nach einem der Ansprüche 1 bis 3, dadurch gekennzeichnet, daß die Kammer (13, 73) teilweise von einer Hülse (17) durchsetzt ist, welche mit der Schlauchleitung (5) in Verbindung steht.

5. Vorrichtung nach Anspruch 4, dadurch gekennzeichnet, daß nach einem Abnehmen des Übertragungskopfes (4) von der Auffangvorrichtung die Hülse (17) zum Abfangen einer Gewebeflüssigkeit mit einer Hülsenöffnung (18) auf eine Anschlußdichtung (23) zu einem Schlauch (22) aufsetzbar ist.

6. Vorrichtung nach Anspruch 5, dadurch gekennzeichnet, daß ein Auffangbehältnis (24) für Gewebeflüssigkeit über den Schlauch (22) mit dem Übertragungskopf (4) in Verbindung steht.

7. Vorrichtung nach wenigstens einem der Ansprüche 1 bis 6, dadurch gekennzeichnet, daß der Übertragskopf (4) mit einer Drehventileinheit (10) verbunden ist.

8. Vorrichtung nach Anspruch 7, dadurch gekennzeichnet, daß mit der Drehventileinheit (10) die Verbindung vom Übertragungskopf (4) zur vakuumerzeugenden Einrichtung (8) herstell- und unterbrechbar ist.

9. Vorrichtung nach Anspruch 7 oder 8, dadurch gekennzeichnet, daß die Drehventileinheit (10) aus einer Grundscheibe (30) und einer mit dem Übertragungskopf (4) verbundenen Drehscheibe (31) besteht, welche zur Grundscheibe (30) hin eine Achse (32) besitzt.

10. Vorrichtung nach Anspruch 9, dadurch gekennzeichnet, daß die Grundscheibe (30) einen Anschluß (33) zu der Leitung (9) zu der Vakuumpumpe (8) hin besitzt.

11. Vorrichtung nach Anspruch 9 oder 10, dadurch gekennzeichnet, daß die Drehscheibe (31) von zumindest zwei Bohrungen (36, 37) durchsetzt ist, welche alternierend mit dem Anschluß (33) bzw. einer Bohrung (34) in der Grundscheibe (30) in Verbindung bringbar sind.

12. Vorrichtung nach Anspruch 12, dadurch gekennzeichnet, daß die Bohrung (36) über das Leitungsstück (11) mit dem Übertragungskopf (4) verbunden ist.

13. Vorrichtung nach Anspruch 11 oder 12, dadurch gekennzeichnet, daß die Bohrung (37) über einen Schlauch (26) mit dem Auffangbehältnis (24) verbunden ist.

14. Vorrichtung nach wenigstens einem der Ansprüch 7 bis 13, dadurch gekennzeichnet, daß der Übertragungskopf (4) über einen Drehhebel (3) mit der Drehventileinheit (10) verbunden ist.

15. Vorrichtung nach Anspruch 14, dadurch gekennzeichnet, daß der Drehhebel (3) von einer Längsbohrung (12) durchsetzt ist, welche einerseits mit dem Leitungsstück (11) in Verbindung steht und andererseits in die im Übertragungskopf (4) gebildete Kammer (13) einmündet.

16. Vorrichtung nach wenigstens einem der Ansprüche 1 bis 15, dadurch gekennzeichnet, daß die Auffangvorrichtung (7) aus einem Stützkörper (38) und einem diesem aufgesetzten Drehteller (39) besteht.

17. Vorrichtung nach Anspruch 16, dadurch gekennzeichnet, daß im Stützkörper (38) ein Antrieb für den Drehteller (39) um eine Drehachse (40) vorgesehen ist.

18. Vorrichtung nach Anspruch 16 oder 17, dadurch gekennzeichnet, daß zur Lagebestimmung des Drehtellers (39) zwischen diesem und dem Stützkörper (38) federgelagerte Rastkugeln (53) vorgesehen sind.

19. Vorrichtung nach einem der Ansprüche 16 bis 18, dadurch gekennzeichnet, daß der Drehteller (39) aus einer Tragscheibe (43) und einer Deckscheibe (46) besteht, zwischen denen Kragen (45) von Reagenzgläsern (6) gehalten sind.

20. Vorrichtung nach wenigstens einem der Ansprüche 16 bis 19, dadurch gekennzeichnet, daß der Drehteller (39) einer mit dem Stützkörper (38) verbundenen Antriebswelle (41) lösbar aufgesetzt ist.

21. Vorrichtung nach Anspruch 2, dadurch gekennzeichnet, daß eine Bohrung (29, 83) zum Belüften der Kammer (13, 73) von einem Hebel (27), einer Schubhülse (85) mit Dichtung (84) od. dgl. überdeckt ist.

22. Vorrichtung nach einem der Ansprüche 2 oder 21, dadurch gekennzeichnet, daß die Kammer (13, 73) teilweise von einem Röhrchen (78) durchsetzt ist, welches mit der Schlauchleitung (5) in Verbindung steht.

23. Vorrichtung nach wenigstens einem der Ansprüch 2, 21 oder 22, dadurch gekennzeichnet, daß die Schlauchleitung (5) in ein Anschlußstück (75) einmündet, welches eine Rastverbindung mit dem Übertragungskopf (4a) eingeht.

24. Vorrichtung nach Anspruch 23, dadurch gekennzeichnet, daß dem Übertragungskopf (4a) eine segmentierte Ringhülse (79) angeformt ist, in welche eine Kugel (77) des Anschlußstückes (75) einsetzbar ist.

25. Vorrichtung nach Anspruch 24, dadurch gekennzeichnet, daß sich die Kugel (77) über eine Dichtung (82) gegen die Ringhülse (79) bzw. einen Kugelsitz (81) abstützt.

26. Vorrichtung nach wenigstens einem der Ansprüche 1, 2 oder 21 bis 25, dadurch gekennzeichnet, daß der Übertragungskopf (4a) an einen Schlitten (58) angeordnet ist, der mittels eines Antriebes entlang einer Führungsschiene (61) bewegbar und seine Position der Auffangvorrichtung (57) automatisch zugeordnet ist.

## Claims

1. Device for recovering a tissue sample, tissue fluid or the like, which is detached and/or aspirated by a surgical instrument, there being a tubing (5) between the surgical instrument and a collecting device (7, 57), which tubing has a vacuum-producing apparatus (8) associated therewith, characterised in that the tubing (5) terminates in a chamber (13, 73) in a transmission head (4, 4a) which rests on the collecting device (7, 57) with a ring seal (15), the chamber (13, 73) communicating with the vacuum-producing apparatus (8) via a line (9, 11, 12).

2. Device according to claim 1, characterised in that the chamber (13, 73) can be ventilated.

3. Device according to claim 2, characterised in that a borehole (29, 83) for ventilating the chamber (13, 73) is covered by a lever (27).

4. Device according to one of claims 1 to 3, characterised in that the chamber (13, 73) is partially traversed by a sleeve (17) which communicates with the tubing (5).

5. Device according to claim 4, characterised in that, after the transmission head (4) has been removed from the collecting device, the sleeve for recovering a tissue fluid with a sleeve opening (18), is mountable on a connection seal (23) belonging to a tubing (22).

6. Device according to claim 5, characterised in that a collecting container (24) for tissue fluid communicates with the transmission head (4) via the tubing (22).

7. Device according to at least one of claims 1 to 6, characterised in that the transmission head (4) is connected to a rotary valve unit (10).

8. Device according to claim 7, characterised in that the connection from the transmission head (4) to the vacuum-producing apparatus (8) can be made and broken with the rotary valve unit (10).

9. Device according to claim 7 or 8, characterised in that the rotary valve unit (10) comprises a base disc (30) and a rotating disc (31), which is connected to the transmission head (4) and has an axis (32) extending towards the base disc (30).

10. Device according to claim 9, characterised in that the base disc (30) has a connection (33) with the line (9) leading to the vacuum pump (8).

11. Device according to claim 9 or 10, characterised in that the rotating disc (31) is traversed by at least two boreholes (36, 37) which can alternatingly be made to connect with the connection (33) or respectively with a borehole (34) in the base disc (30).

12. Device according to claim 12, characterised in that the borehole (36) is connected to the transmission head (4) by the line piece (11).

13. Device according to claim 11 or 12, characterised in that the borehole (37) is connected to the collecting container (24) via a tubing (26).

14. Device according to at least one of claims 7 to 13, characterised in that the transmission head (4) is connected to the rotary valve unit (10) via a rotating lever (3).

15. Device according to claim 14, characterised in that the rotating lever (3) is traversed by a longitudinal borehole (12), which communicates at one end with the line piece (11) and terminates at the other end in the chamber (13) formed in the transmission head (4).

16. Device according to at least one of claims 1 to 15, characterised in that the collecting device (7) comprises a supporting body (38) and a rotating plate (39) mounted thereon.

17. Device according to claim 16, characterised in that a drive for driving the rotating plate (39) about an axis of rotation (40) is provided in the supporting body (38).

18. Device according to claim 16 or 17, characterised in that spring-mounted locking balls (53) are provided between the rotating plate (39) and the supporting body (38), in order to determine the position the rotating plate (39).

19. Device according to one of claims 16 to 18, characterised in that the rotating plate (39) comprises a supporting disc (43) and a covering disc (46), between which discs collars (45) of test tubes (6) are retained.

20. Device according to at least one of claims 16 to 19, characterised in that the rotating plate (39) is detachably mounted on a drive shaft (41), which is connected to the supporting body (38).

21. Device according to claim 2, characterised in that a borehole (29, 83) for ventilating the chamber (13, 73) is covered by a lever (27), a sliding sleeve (85) having seal (84), or the like.

22. Device according to one of claims 2 or 21, characterised in that the chamber (13, 73) is partially traversed by a small tube (78), which communicates with the tubing (5).

23. Device according to at least one of claims 2, 21 or 22, characterised in that the tubing (5) terminates in a connection piece (75), which forms a locking connection with the transmission head (4a).

24. Device according to claim 23, characterised in that a segmented ring sleeve (79) is configured to fit on the transmission head (4a), into which sleeve a ball (77) of the connection piece (75) is insertable.

25. Device according to claim 24, characterised in that the ball (77) is supported against the ring sleeve (79), or respectively a ball seat (81), via a seal (82).

26. Device according to at least one of claims 1, 2 or 21 to 25, characterised in that the transmission head (4a) is disposed on a carriage (58), which is displaceable along a guide rail (61) by means of a drive, and its position is automatically associated with the collecting device (57).

## Revendications

1. Dispositif pour recueillir des échantillons de tissus, du liquide lymphatique ou autres, prélevés à l'aide d'un instrument chirurgical et/ou aspirés, selon lequel, entre l'instrument chirurgical et un dispositif de collecte (7, 57), se trouve un tuyau (5) auquel est associée une installation (8) créant du vide, caractérisé en ce que le tuyau (5) débouche dans une chambre (13, 73) d'une tête de transmission (4, 4a) s'appuyant par un joint annulaire (15) du dispositif de collecte (7, 57), la chambre (13, 73) communiquant par une conduite (9, 11, 12) à l'installation (8) créant le vide.

2. Dispositif selon la revendication 1, caractérisé en ce que la chambre (13, 73) peut être ventilée.

3. Dispositif selon la revendication 2, caractérisé par un perçage (29, 83) pour ventiler la chambre (13, 73) est recouvert un levier (27).

4. Dispositif selon l'une des revendications 1 à 3, caractérisé en ce que la chambre (13, 73) est traversée partiellement par un manchon (17) communiquant avec le tuyau (5).

5. Dispositif selon la revendication 4, caractérisé en ce qu'après enlèvement de la tête de transmission (4) par rapport au dispositif de collecte, le manchon (17) peut être mis avec son ouverture (18) sur un joint de raccordement (23) d'un tuyau (22) pour recueillir un liquide lymphatique.

6. Dispositif selon la revendication 5, caractérisé en ce qu'un réceptacle de collecte (24) pour le liguide lymphatique est relié à la tête de transmission (4) par le tuyau (22).

7. Dispositif selon au moins l'une des revendications 1 à 6, caractérisé en ce que la tête de transmission (4) est reliée à un ensemble à boisseau tournant (10).

8. Dispositif selon la revendication 7, caractérisé en ce que l'ensemble à boisseau tournant (10) permet d'établir ou de couper la liaison entre la tête de transmission (4) et l'installation (8) créant le vide.

9. Dispositif selon la revendication 7 ou 8, caractérisé en ce que l'ensemble à boisseau tournant (10) se compose d'un disque de base (30) et d'un disque tournant (31) relié à la tête de transmission (4), ce disque ayant un axe (32) vers le disque de base (30).

10. Dispositif selon la revendication 9, caractérisé en ce que le disque de base (30) comporte un branchement (33) pour la conduite (9) vers la pompe à vide (8).

11. Dispositif selon la revendication 9 ou 10, caractérisé en ce que le disque tournant (31) est traversé par au moins deux perçages (36, 37) qui peuvent être mis en communication en alternance avec le branchement (33) ou un perçage (34) du disque de base (30).

12. Dispositif selon la revendication 11, caractérisé en ce que le perçage (36) est relié à la tête de transmission (4) par un morceau de conduite (11).

13. Dispositif selon la revendication 11 ou 12, caractérisé en ce que le perçage (37) est relié par un tuyau (26) au réceptacle de collecte (24).

14. Dispositif selon l'une des revendications 7 à 13, caractérisé en ce que la tête de transmission (4) est reliée par un levier tournant (3) à l'ensemble à boisseau tournant (10).

15. Dispositif selon la revendication 14, caractérisé en ce que le levier tournant (3) est traversé par un perçage longitudinal (12) communiquant d'une part au morceau de tuyau (11) et d'autre part avec une chambre (13) formée dans la tête de transmission (4).

16. Dispositif selon au moins l'une des revendications 1 à 15, caractérisé en ce que le dispositif de collecte (7) se compose d'un organe d'appui (38) et d'un plateau tournant (39) prévu sur l'organe d'appui.

17. Dispositif selon la revendication 16, caractérisé en ce que l'organe d'appui (38) est muni d'un moyen d'entraînement pour tourner le plateau tournant (39) autour d'un axe de rotation (40).

18. Dispositif selon la revendication 16 ou 17, caractérisé par des billes d'encliquetage (53) montées à ressort pour définir la position du plateau tournant (39) par rapport à l'organe d'appui (38).

19. Dispositif selon l'une des revendications 16 à 18, caractérisé en ce que le plateau tournant (39) se compose d'un disque de support (43) et d'un disque de couverture (46) entre lesquels sont maintenues les collerettes (45) des éprouvettes (6).

20. Dispositif selon au moins l'une des revendications 16 à 19, caractérisé en ce que le plateau tournant (39) est monté de manière amovible sur un axe d'entraîne. ment (41) relié à l'organe d'appui (38).

21. Dispositif selon la revendication 2, caractérisé par un percage (29, 83) pour ventiler la chambre (13, 73) qui est ouvert par un levier (27), un manchon coulissant (85) avec un joint d'étanchéité (84) ou moyen analogue.

22. Dispositif selon l'une des revendications 2 ou 21, caractérisé en ce que la chambre (13, 73) est traversée partiellement par un petit tube (78) communiquant avec le tuyau (5).

23. Dispositif selon au moins l'une des revendications 2, 21 ou 22, caractérisé en ce que le tuyau (5) débouche dans une pièce de raccordement (75) avec un moyen d'encliquetage pour la tête de transfert (4a).

24. Dispositif selon la revendication 23, caractérisé en ce qu'un manchon annulaire segmenté (79) est formé sur la tête de transmission (4a) recevant une bille (77) de la pièce de raccordement (75).

25. Dispositif selon la revendication 24, caractérisé en ce que la bille (77) s'appuie par un joint (82) contre le manchon annulaire (79) ou un siège à bille (81).

26. Dispositif selon au moins l'une des revendications 1, 2 ou 21 à 25, caractérisé en ce que la tête de transmission (4a) est montée sur un chariot (58) déplacé par un moyen d'entraînement le long d'un rail de guidage (61) et dont la position est associée automatiquement au dispositif de collecte (57).
